(19) **Europäisches Patentamt / European Patent Office / Office européen des brevets**

(11) **EP 4 466 537 B1**

(12) **EUROPÄISCHE PATENTSCHRIFT**

(45) Veröffentlichungstag und Bekanntmachung des Hinweises auf die Patenterteilung:
**08.04.2026  Patentblatt 2026/15**

(21) Anmeldenummer: **23701668.8**

(22) Anmeldetag: **20.01.2023**

(51) Internationale Patentklassifikation (IPC):
**G01N 33/24** (2006.01)     **G01N 13/04** (2006.01)

(52) Gemeinsame Patentklassifikation (CPC):
**G01N 33/246; G01N 13/04**

(86) Internationale Anmeldenummer:
**PCT/EP2023/051365**

(87) Internationale Veröffentlichungsnummer:
**WO 2023/139209 (27.07.2023 Gazette 2023/30)**

(54) **TENSIOMETER UND VERFAHREN ZUR BESTIMMUNG EINES RÄUMLICH GEMITTELTEN WASSERPOTENTIALS IN EINEM TESTKÖRPER**

TENSIOMETER AND METHOD FOR DETERMINING A SPATIALLY AVERAGED WATER POTENTIAL IN A TEST PIECE

TENSIOMÈTRE ET PROCÉDÉ DE DÉTERMINATION D'UN POTENTIEL D'EAU MOYENNÉ DANS L'ESPACE DANS UNE ÉPROUVETTE

(84) Benannte Vertragsstaaten:
**AL AT BE BG CH CY CZ DE DK EE ES FI FR GB GR HR HU IE IS IT LI LT LU LV MC ME MK MT NL NO PL PT RO RS SE SI SK SM TR**

(30) Priorität: **21.01.2022  DE 102022200685**

(43) Veröffentlichungstag der Anmeldung:
**27.11.2024  Patentblatt 2024/48**

(73) Patentinhaber: **Membran Tech GmbH**
**03042 Cottbus (DE)**

(72) Erfinder:
• **LAZIK, Detlef**
  **06198 Salzatal (DE)**
• **DE ROOIJ, Gerrit**
  **06120 Halle (DE)**

(74) Vertreter: **Patentanwälte Bressel und Partner mbB**
**Potsdamer Platz 10**
**10785 Berlin (DE)**

(56) Entgegenhaltungen:
**WO-A1-2014/201442     DE-A1- 19 714 586**
**US-A- 4 891 968     US-A- 5 005 403**

• **DURNER WOLFGANG ET AL: "73: Soil Water Potential Measurement", ENCYCLOPEDIA OF HYDROLOGICAL SCIENCES, 2005, XP093030858, Retrieved from the Internet <URL:https://citeseerx.ist.psu.edu/document?repid=rep1&type=pdf&doi=ba26351a2c31f18f721032be9f1e712eae7929b2> [retrieved on 20230310]**

## Beschreibung

Die Erfindung betrifft ein Tensiometer und ein Verfahren zur Bestimmung eines räumlich gemittelten Wasserpotentials in einem Testkörper.

## Hintergrund

Pflanzen müssen das Wasserpotential des Bodens überwinden, um diesem Wasser zu entziehen. Dieses Wasserpotential setzt sich aus dem gravimetrischen Potential, dem Matrixpotential und dem osmotischen Potential zusammen. Unter humiden ungesättigten Verhältnissen wird das lokal vorhandene Wasserpotential im Allgemeinen gleich dem Matrixpotential gesetzt - das osmotische Potential kann im Allgemeinen vernachlässigt werden. In ariden Böden kann die Konzentration gelöster Salze insbesondere infolge der Verdunstung des Bodenwassers im oberflächennahen Boden einen erhöhten Beitrag des osmotischen Potentials zum Wasserpotential bewirken, der nicht mehr vernachlässigt werden kann. Äußere Bedingungen wie Niederschlag oder Beregnung, Luftfeuchte, Temperatur, Verdunstung und lokal unterschiedliche innere Quell-/Senken-Phänomene im Boden, wie der schwerkraftbedingte Wasserentzug, der Wasserentzug über die Pflanzenwurzel oder die Bindung von Wasser an hygroskopische Substanzen (häufig Salze) führen ständig zu lokalen Änderungen des Wasserpotentials, die wiederum zeiträumlich variierende Wasserflüsse bewirken.

## Stand der Technik

Die Messung des Wasserpotentials erfolgt durch Tensiometer die dem Messprinzip nach in direkt und indirekt messende Tensiometer unterschieden werden. Die direkte Messung basiert auf einem wassergefüllten Hohlraum der von einer porösen, kapillar mit dem Boden in Verbindung stehenden Filterkerze gekapselt und mit einem Drucksensor ausgestattet ist. Bedingt das außen anliegende Wasserpotential den Entzug von Wasser aus dem Hohlraum, so führt dies zu einem Abfall des Drucks in diesem Tensiometertyp. Die dabei entstehende Druckdifferenz zum atmosphärischen Druck (auch als Saugspannung bezeichnet) entspricht im Gleichgewicht dem Wasser- bzw. Matrixpotential. Bei den indirekten Verfahren erfolgt eine Gleichgewichtseinstellung des Wasserpotentials im Boden zu einem separaten hygroskopischen Medium, in dem die Wasseraktivität oder das Wasserpotential in geeigneter Weise messbar ist. Messmethoden basieren beispielsweise auf der Änderung der Kapazität eines aus dem Medium bestehenden Dielektrikums eines Kondensators, der elektrischen Leitfähigkeit des Mediums, der Ausbreitung von Wärmeimpulsen durch das Medium oder auch des Drucks in diesem Medium.

Insbesondere unter trockenen Bedingungen, also dort, wo der Bedarf an Bewässerungswasser an die Grenzen des unter ökonomischen Bedingungen verfügbaren Wassers stößt, sind Bewässerungsstrategien gefragt, die Pflanzen zuverlässig vor dem Erreichen des permanenten Welkepunktes (Pflanzen werden irreparabel geschädigt) mit Wasser versorgen. Im Bereich des permanenten Welkepunktes können die Wasserpotentiale im Extremfall -1.5 MPa bis -2.5 MPa erreichen. Die Wurzeln saugen nun, bildlich, mit einer dem Unterdruck äquivalenten hängenden Wassersäule der Höhe $\Delta h = \rho_w g \psi_w$ (Wasserdichte $\rho_w$, Gravitationskonstante g, Wasserpotential $\Psi_w$) von 15000 ... 25000 cmWs (cm Wassersäule) am restlichen, noch in Kapillaren und auf Oberflächen (Wasserfilme, Zwickelfüllungen) physikalisch gebundenen Wasser. In direkt messenden Tensiometern würde das Wasser unter diesen Bedingungen sieden, sie sind in diesen Bereichen daher nicht mehr einsatzfähig. Bereits bei einer Saugspannung von 700 bis 800 cmWs bilden sich in diesem Typ Tensiometer Wasserdampfblasen, die eine Messung des außen anliegenden Wasserpotentials unmöglich machen. Indirekt messende Tensiometer, beispielsweise das Polymertensiometer, spielen in diesem Bereich ihre Stärke aus. Die mit einem Drucksensor ausgestattete Messkammer dieses Tensiometertyps ist mit einem hygroskopischen Polymer befüllt, das über eine mikroporöse Membran, beispielsweise mikroporöses Aluminiumoxyd, durch eine beispielsweise bis zu 15 bar druckstabile poröse Kerze an das außen vorhandene Wasser ankoppelt. Als hygroskopische Polymere werden in der Literatur Stoffe wie Polyethylenglycol (PEG), Polyacrylamid (Praestol 2500) oder Polysacharid (Dextran 500), angegeben, welche aufgrund ihrer Molekülgröße die mikroporöse Membran nicht durchdringen können, andererseits jedoch eintretendes Wasser binden und dabei dessen Aktivität reduzieren, sodass der Umgebung weiteres Wasser entzogen wird. Dabei entsteht ein Überdruck (osmotischer Druck) gegenüber dem Druck des Wassers in der Umgebung des Tensiometers. Im Gleichgewicht der chemischen Potentiale des Wassers im Polymer, bestimmt durch dessen osmotisches Potential und im Boden, bestimmt durch dessen Wasserpotential, verschiebt sich dieser Überdruck proportional mit dem außen anliegenden Wasserpotential. Fällt das äußere Wasserpotential, entzieht der Boden dem Polymertensiometer Wasser und der Fluiddruck im Polymer sinkt und umgekehrt. Solange der Druck im Polymertensiometer größer ist, als der Wasserdampfdruck, wird die Bildung von Wasserdampfblasen verhindert. Dieser, den Messbereich begrenzende Druck ist durch die Art des Polymers bestimmt und fällt näherungsweise mit dem osmotischen Potential des im Wasser übersättigten Polymers zusammen. US5005403 offenbart eine Bestimmung einer Konzentration in Lösungen mit einer Messung eines differentiellen osmotischen Drucks über eine permeable Membran.

## Probleme des Polymertensiometers

Nach der Kalibration des Polymertensiometers gegen eine frei bewegliche Wasserphase bei Luftdruck (im Folgenden als Standardbedingungen bezeichnet) und der Berücksichtigung von Temperaturabhängigkeiten kann das Wasserpotential des Bodens bestimmt werden. Prinzipbedingt ist dieses Tensiometerprinzip insbesondere für tiefe (betragsmäßig große) Wasserpotentiale und große Messbereichsweiten geeignet, da der Bezugspunkt der Messanordnung durch das osmotische Potential des Polymers festgelegt wird. Wird dieses näherungsweise durch das Wasserpotential im Testkörper kompensiert, nähert sich der Druck im Tensiometer dem Nullpunkt (nahe des Wasserdampfdrucks).

Die notwendige Zeit zur Gleichgewichtseinstellung begrenzt das Volumen der mit dem Polymer befüllten Messkammer. Deshalb wurden Polymertensiometer mit deutlich minimiertem Messkammervolumen bei maximierter Austauschfläche, beispielsweise mit Messkammern mit einer Kammerhöhe von 1.1 bis 2.5 mm bei einem Radius von 8.45 mm und konischen Austauschflächen im Bereich von 167 ... 260 mm$^2$, entwickelt. Was hinsichtlich der Minimierung der Einstellzeit als wichtiger Optimierungsschritt anzusehen ist, bedingt aus Sicht der Feldanwendung den lokalen Bezug der Messung.

Hinsichtlich einer effizienten Steuerung beispielsweise einer Bewässerungsanlage wäre jedoch ein Wasserpotentialsensor wünschenswert, der über die lokal unterschiedlichen Wasserpotentiale mittelt. Insbesondere wäre die Messbarkeit dieses mittleren Wasserpotentials über einen lateralen Bereich wünschenswert, dessen Größe beispielsweise genügt, um über unterschiedlich stark durchwurzelte Bodenbereiche repräsentativ zu mitteln. Ein solcher, räumlicher Mittelungsbereich wird durch die bodenabhängige Dimension des sogenannten Pedons charakterisiert, einem Bodenausschnitt mit einem Volumen, dass hinreichend groß gewählt wird, so dass die relevanten Eigenschaften des Bodens standortabhängig statistisch ausreichend gemittelt (repräsentativ) eingehen. Häufig wird dieser Pedon als Bodenausschnitt mit einer lateralen Erstreckung von etwa 1 m angesehen, woran sich häufig der Durchmesser von Lysimetern orientiert. Abhängig vom Bodentyp, aber auch von dessen Pflanzenbestand wird die tatsächlich nötige Mittelungslänge (Pedon) zur repräsentativen Bestimmung des Wasserpotentials insbesondere in einem Bereich von 1 dm bis zu mehr als 10 m liegen.

## Aufgabe

Der Erfindung liegt die Aufgabe zugrunde, ein robustes, indirekt messendes Tensiometer und ein Verfahren zu schaffen, dass eine repräsentative Bestimmung von Wasserpotential oder Matrixpotential in einem heterogen zusammengesetzten Testkörper ermöglicht, wie dies beispielsweise ein pedonskaliger Ausschnitt eines Bodenkörpers darstellt. Dabei sollen das Tensiometer und das darauf aufbauende Verfahren sowohl für feuchte (humide) als auch für trockene Böden (arider Klimabereiche) ermöglichen, die lokal unterschiedlich hohen Wasserpotentiale repräsentativ gemittelt zu erfassen. Um die Messgenauigkeit und den Messbereich optimal an die jeweilige Anwendung anzupassen soll ferner insbesondere: i) der Bezugspunkt der Messanordnung auf das Wasserpotential der freien Wasseroberfläche bei Luftdruck (Standardbedingungen) verlegt werden, da hier naturgemäß die größte Genauigkeit erreicht werden muss, ii) problemabhängig sollen auch weitere Bezugspotentiale einstellbar sein und iii) die Weite des Messbereichs soll problemabhängig durch den Nutzer einstellbar sein bzw. nutzungsabhängig vorgebbar sein.

## Erfindungsgemäße Umsetzung

Die Aufgabe wird durch ein Tensiometer mit den Merkmalen des Patentanspruchs 1 und ein Verfahren mit den Merkmalen des Patentanspruchs 15 gelöst. Vorteilhafte Ausgestaltungen der Erfindung ergeben sich aus den Unteransprüchen.

Das im Folgenden eingeführte indirekt messende Tensiometer zur Messung von Wasserpotential oder Matrixpotential ist mit einem Referenzsystem ausgestattet und basiert auf mindestens zwei nichtporösen, semipermeablen Membranschläuchen aus fallweise geeigneten Polymeren, die mit osmotischen Lösungen befüllt und verschlossen sind. Bedingt durch die Art der Membranen können Stoffe diese nur diffusiv überwinden. Eine Druckdifferenz zwischen den osmotischen Lösungen wird mittels geeigneter Drucksensoren gemessen. Ferner wird ein Verfahren eingeführt, nachdem die mittels des Tensiometers (nach einer der in dieser Offenbarung beschriebenen Ausführungsformen) erfasste Druckdifferenz ein über die Länge des Membranschlauchs gemitteltes Maß liefert, dass bei geeigneter Wahl von Bezugspotential und Membranmaterial mit dem räumlich gemittelten Wasserpotential oder dem räumlich gemittelten Matrixpotential des Wassers im Testkörper korrespondiert. Tensiometer und Verfahren ermöglichen es somit wie folgend ausgeführt, das Wasserpotential oder das Matrixpotential im Testkörper, beispielsweise einem pedonskaligen Bodenkörper, räumlich gemittelt zu erfassen.

Insbesondere wird ein Tensiometer geschaffen, umfassend eine als Potentialzelle bezeichnete Messzelle wählbarer Länge, die eine mit einer schlauchförmigen semipermeablen Membran ummantelte verschließbare Messkammer umfasst und in einen Testkörper eingebaut werden kann; ein Referenzsystem umfassend eine Referenzkammer und eine als Referenzzelle bezeichnete Messzelle wählbarer Länge, die eine mit einer schlauchförmigen semipermeablen Membran ummantelte verschließbare Messkammer umfasst, wobei die Referenzzelle in die Referenzkammer des Referenzsystems integriert ist, und wobei die Referenzkammer eine osmotische Referenzlösung enthält oder mit einer solchen befüllbar ist, wobei die Potentialzelle und die Referenzzelle mit einer osmotischen Lösung befüllt oder befüllbar sind; und eine Druckmesseinrichtung, die dazu eingerichtet ist, zumindest eine Druckdifferenz zwischen den Messkammern der Potentialzelle und der Referenzzelle zu messen.

Ferner wird insbesondere ein Verfahren zur Bestimmung eines räumlich gemittelten Wasserpotentials in einem Testkörper zur Verfügung gestellt, wobei mittels des Tensiometers nach einer der beschriebenen Ausführungsformen zumindest die Druckdifferenz zwischen den Messkammern der Potentialzelle und der Referenzzelle erfasst wird, die aufgrund ihrer Anordnung innerhalb der Referenzkammer des Referenzsystems den Bezug zu einem wählbaren Bezugspotential gestattet, wobei ausgehend von der erfassten Druckdifferenz in Abhängigkeit von der verwendeten semipermeablen Membran und des Bezugspotentials das über die Länge der Potentialzelle gemittelte Wasser- und/oder Matrixpotential im Testkörper bestimmt und als Messwert bereitgestellt wird.

## Bestandteile des Tensiometers und deren Begriffe

Das Tensiometer umfasst eine Potentialzelle und ein Referenzsystem, und ferner insbesondere ein Tensiometerboard und auslegungsabhängig geeignete Gehäusekomponenten (die Bezugszeichen werden mit Bezug auf die Figuren angegeben).

Die Potentialzelle umfasst einen Membranschlauch, dessen äußere Oberfläche zur Messung des Wasserpotentials in Kontakt mit einem Substrat im Testkörper steht und dessen innere Oberfläche, insbesondere radial, eine Messkammer begrenzt.

Das Referenzsystem umfasst eine mit einer Referenzlösung befüllte Referenzkammer, in die eine Referenzzelle integriert ist.

Die Referenzzelle umfasst einen Membranschlauch, dessen äußere Oberfläche zur Messung eines Bezugspotentials in Kontakt mit der Referenzlösung steht und dessen innere Oberfläche, insbesondere radial, eine weitere Messkammer begrenzt.

Die Messkammern sind bzw. werden insbesondere durch die jeweiligen Membranschläuche ausgebildet.

Potentialzelle und Referenzzelle sind insbesondere als radialsymmetrische Messzellen ausgebildet, die über geeignete Messeinrichtungen und über funktionale Elemente (zur Volumenreduktion und mechanischen Stabilisierung verfügen können. Die insbesondere mit Ventilen verschließbaren Messkammern beider Messzellen sind mit gleichen osmotischen Lösungen befüllt.

Das Tensiometerboard enthält eine integrierte elektronische Beschaltung zur Datenerfassung, Bearbeitung, Auswertung, Speicherung und/oder Ausgabe sowie auslegungsabhängig geeigneten Anschlussmöglichkeiten.

Das Tensiometer wird von einer internen oder externen Spannungsquelle mit Energie versorgt.

## Funktionsprinzip

Nachfolgend wird das Funktionsprinzip des Tensiometers beispielhaft unter Zugrundelegung von insbesondere baugleichen Messzellen näher erläutert. Die Potentialzelle soll äußerlich einem Testkörper, insbesondere dem Boden, ausgesetzt sein. Die Referenzzelle soll sich in der mit der Referenzsubstanz, z.B. Wasser, befüllten Referenzkammer bei Luftdruck (Standardbedingungen) befinden. Beide Messzellen seien lokal der gleichen äußeren Temperatur ausgesetzt, gegeneinander und vom Außenraum abgeschlossen und enthalten gelöst in Wasser initial gleiche Konzentrationen einer hygroskopischen Substanz. Eine Druckmesseinrichtung erfasst den Differenzdruck $\Delta p$ zwischen den Messkammern von Potentialzelle und Referenzzelle. Alternativ hierzu kann die Druckmesseinrichtung auch die Differenzdrücke der jeweiligen Messkammern $\Delta p_P$ und $\Delta p_R$ zum atmosphärischen Druck erfassen (Index "$P$", bzw. "$R$" für Potential- bzw. Referenzzelle).

Die Messkammern sind radial durch schlauchförmige semipermeable nichtporöse Membranen verschlossen, die vom Wasser nur diffusiv überwunden werden können. Dieser Austausch von Wasser mit der jeweiligen Umgebung erfolgt, bis der Unterschied der chemischen Potentiale des Wassers beidseitig der jeweiligen Membran verschwindet. Das chemische Potential und die Aktivität von Wasser sind im Gleichgewicht analytisch miteinander verknüpft. Während außerhalb die Aktivität des Wassers durch das dort um die Potentialzelle herrschende Matrixpotential $\Psi_m$ und das osmotische Potential der Porenlösung $\psi_\Pi$ bzw. das Bezugspotential $\psi_0$ der als Bezugssystem um die Referenzzelle vorhandenen Referenzlösung bestimmt wird, erfolgt dies innerhalb der beiden Messkammern (Index "$MK$") durch das osmotische Potential $\psi_{MK} = \psi_P = \Psi_R$ der initial gleichen osmotischen Lösungen.

Wässrige Lösungen sind näherungsweise inkompressibel. Daher resultiert aus dem Wasseraustausch für eine ideale, volumenkonstant angenommene Messkammer der Länge L ein sich ändernder osmotischer Druck, der sich ortsabhängig in dieser je nach Lage des äußeren Wasserpotentials einstellen würde. Bei ausreichend kleiner Viskosität der osmotischen Lösung gleicht diese jedoch die lokalen Druckunterschiede in der Messkammer hinreichend schnell aus - ein mittlerer Druck stellt sich über die gesamte Länge der Messkammer ein. Der auf den atmosphärischen Luftdruck bezogene Differenzdruck in der Potentialzelle wird damit:

$$\Delta p_P = \overline{\Psi}_w - \Psi_{MK}, \quad \overline{\Psi}_w = \frac{1}{L}\int_0^L \Psi_w(x)\,dx$$

Statt zu einem lokal bei einem Ort $x_0$ vorhandenen Wasserpotential $\Psi_w(x_0)$, ist der Differenzdruck $\Delta p_P$ somit proportional zu dem, über die Länge der Potentialzelle gemittelten Bodenwasserpotential $\overline{\psi}_w$. Analog lie-

fert der auf den atmosphärischen Luftdruck bezogene Differenzdruck in der Referenzzelle $\Delta p_R = \psi_0 - \psi_{MK}$ und schafft so eine zeitgleiche Referenz zum Bezugspotential $\Psi_0$, beispielsweise zu $\psi_0 = 0$ für Standardbedingungen.

Die Druckdifferenz zwischen beiden als ideal (volumenkonstant) angenommenen Messzellen $\Delta p_P - \Delta p_R = \overline{\psi_w} - \psi_0$ eliminiert in guter Näherung das in den Messkammern herrschende osmotische Potential. Gleichzeitig bewirkt diese Kopplung der beiden Messzellen eine Drehung des Messbereichs, womit der Nullpunkt der Druckdifferenz exakt durch das Bezugspotential definiert ist. Ist das Bezugspotential für Standardbedingungen festgelegt, so ist das im Testkörper vorhandene mittlere Wasserpotential durch $\overline{\psi_w} = \Delta p_P - \Delta p_R$ bestimmt. Wird der Differenzdruck $\Delta p = \Delta p_P - \Delta p_R$ zwischen den beiden Messkammern direkt mittels eines Differenzdrucksensors bestimmt, so ergibt sich das mittlere Wasserpotential nach $\overline{\psi_w} = \Delta p$ bereits unmittelbar aus dieser Messung.

**Bestimmung von Wasser- und Matrixpotential**

Das Wasserpotential kann so beispielsweise für das auf Standardbedingungen festgelegte Bezugspotential gemessen werden. Dafür kommen als nichtporöse Membranmaterialien für die Messzellen für Wasser gut durchlässigen Membranen wie beispielsweise PEBAX 1074, PBT/PEO Block Copolymere, Silikon usw. infrage. In einer Vielzahl von Anwendungen, insbesondere unter humiden Klimabedingungen bestimmt dieses Wasserpotential auch hinreichend genau das Matrixpotential, da das osmotische Potential der Bodenlösung häufig vergleichsweise (betragsmäßig) klein ist.

Unter ariden Klimabedingungen (trockene, warme Böden) können infolge der Verdunstung des Lösungsmittels aufkonzentrierte Salze (Elektrolyte) in der Bodenlösung vorliegen, die ein nicht vernachlässigbares osmotisches Potential bewirken. Um das Matrixpotential zu bestimmen, muss die Wirkung dieses osmotischen Potentials im gemessenen Differenzdruck eliminiert bzw. reduziert werden. Werden zum Aufbau der Messkammern für Wasser, nicht aber für die Elektrolytionen permeable Polymermembranen genutzt, so kann eine Kompensation des osmotischen Potentials näherungsweise durch eine geeignete Verschiebung des Bezugspotentials erfolgen. Dazu muss lediglich das Referenzsystem mit einer zur Bodenlösung osmotisch gleichwertigen oder ähnlichen Referenzlösung befüllt werden. Allerdings variiert die Elektrolytkonzentration in der Bodenlösung in Abhängigkeit von Niederschlag, Verdunstung und Salzinventar. Eine Nachführung des Bezugspotentials, beispielsweise gesteuert über Leitfähigkeitsmessungen in der Boden- und der Referenzlösung wäre möglich, bedeutet aber einen erhöhten mess- und regelungstechnischen Aufwand. Ohne eine solche Nachführung wird das mit einem derart aufgebauten Tensiometer gemessene Matrixpotential somit in Abhängigkeit der Differenz der osmotischen Potentiale der Elektrolyte von Boden- und Referenzlösung variieren.

Die Nutzung einer Ionomermembran zum Aufbau der Messkammern würde im Gegensatz dazu auch Ionen gestatten, die Membran zu durchdringen. Die osmotischen Wirkungen der Elektrolyte beidseitig der Potentialzelle heben sich dann näherungsweise auf. Wird das Referenzpotential durch Standardbedingungen festgelegt, so kann das Matrixpotential nun exakt bestimmt werden.

Potenziell geeignete Ionomere sind z.B. Nafion (DuPont), Flemion™ (Asahi Glas Co. Ltd) und Dow-Membrane® (Dow Chemical).

Nafion ("General Information on Nafion® Membrane for Electrolysis", Product Information, Bulletin 97-01, Rev. 04/2006), ein sulfoniertes Tetrafluorethylen (PTFE)-Polymer, ist für Wasser (und andere polare Stoffe) gut durchlässig sowie für Kationen, ist einsetzbar für Temperaturen bis zu 190°C und chemisch äußerst stabil. Das nur geringfügig dehnbare Nafion kann abhängig von dessen Formulierung außerordentlich hohen mechanischen Spannungen widerstehen (vgl. Produktinformation), was hinsichtlich der vorliegenden Anwendung bedeutsam ist. Berücksichtigt man die Druckstabilität von Nafion-Membranschläuchen bis hin zu 16 bar, so resultiert für ein mit diesem Werkstoff aufgebautes Tensiometer ein breiter möglicher Messbereich, innerhalb dessen die Membranschläuche mechanisch nicht gegen den auftretenden Überdruck abgestützt werden müssen.

Für Nafion als Membranmaterial würden Kationen bevorzugt in die Messkammer der Potentialzelle diffundieren gefolgt von Anionen. Damit können entsprechend aufgebaute und mit einer geeigneten osmotischen Lösung befüllte Messzellen, wenn auch zeitversetzt, den gewünschten Ausgleich der außen vorhandenen Elektrolytkonzentration ermöglichen. Während des Konzentrationsausgleichs tritt jedoch wiederum ein osmotischer Differenzdruck auf, der von den Unterschieden in den Elektrolytkonzentrationen beidseitig der Membran abhängt.

Die bevorzugte Diffusion von Kationen durch die Nafionmembran bedingt andererseits eine Ladungstrennung. Infolge der Anreicherung von Kationen in der Messkammer baut sich hier ein positiver Ladungsüberschuss auf begleitet von einem negativen Ladungsüberschuss in der äußeren Umgebung der Membran. Der resultierende elektrochemische Potentialunterschied $\Delta\varphi$ bewirkt eine, der konzentrationsgetriebenen Diffusion, entgegengesetzt gerichtete Migration der Kationen. Nach der Nernst-Planck-Gleichung kann die resultierende Stoffstromdichte $\dot{n}_j$ durch die Membran als Überlagerung von Diffusion und Migration $\dot{n}_j = -D_j(\nabla c_j - F z_j c_j/(RT) \nabla\varphi)$ beschrieben werden, worin $c_j$ die Konzentration des Kations $j$ der Ladung $z_j$ ist und $D_j$ dessen Diffusionskoeffizient in der Membran. $F$ ist die Faraday-Konstante, $R$ die allgemeine Gaskonstante und $T$ die Temperatur. Für vernachlässigbar kleine Stoffströme $(\dot{n}_j \to 0 \, \forall \, j)$, entsteht zwischen der Differenz der Kationenkonzentration und der des elektrochemischen Potentials beidseitig der Membran eine in erster Näherung proportionale Abhängigkeit $\Delta\varphi \sim \Sigma\Delta c_i /(z_i c_i)$.

Die Gesamtheit an Ionen außerhalb und innerhalb der Messkammer unterliegt der Bedingung der Ladungsneutralität. Eine in der Messkammer aufgebaute positive Überschussladung muss deshalb betragsgleich zu der außerhalb resultierenden negativen Überschussladung sein. Beide Überschussladungen bauen so eine von Art und Konzentration der Elektrolyte abhängige elektrische Spannung $U$ über die Membran auf.

Eine geeignete Messung dieser Spannung ermöglicht zum einen den Nachweis über ausgeglichene Elektrolytkonzentrationen beidseitig der Membran, da die Spannung dann verschwinden muss. Unter Zugrundelegung einer Kalibration der Spannung gegen den resultierenden osmotischen Druck in der Potentialzelle existiert jedoch darüber hinaus die Möglichkeit, den Effekt fluktuierender osmotischer Potentialdifferenzen auf die Messung des Matrixpotentials durch Reduktion bzw. Eliminierung des spannungsabhängigen Drucks vom gemessenen Differenzdruck $\Delta p$ auszugleichen. In einer Ausführungsform ist daher vorgesehen, dass das Tensiometer eine Spannungsmesseinrichtung zur Messung einer elektrischen Spannung zwischen der Innenseite und der Außenseite der Membran der Potentialzelle aufweist.

In einer Ausführungsform des Verfahrens ist entsprechend vorgesehen, dass das Matrixpotential $\overline{\psi}_m$ im Testkörper in mit Ionomermembranen ausgestatteten Messzellen ermittelt wird, indem die elektrische Spannung U zwischen der Innenseite und der Außenseite der Membran der Potentialzelle mittels der Spannungsmesseinrichtung erfasst wird, die nach Kalibration ermöglicht, einen Druck zu ermitteln, der durch elektrolytkonzentrationsabhängige osmotische Potentialunterschiede beidseitig der Membran bedingt ist, und diesen aus der gemessenen Druckdifferenz $\Delta p$ zu eliminieren und so das Matrixpotential $\overline{\psi}_m$ zu bestimmen.

## Kompensation der druckabhängigen Ausdehnung nichtidealer Messkammern

Für nichtideale Messkammern kann es infolge der druckabhängigen elastischen Ausdehnung der Messkammern zu einer Verdünnung der osmotischen Lösung kommen, die im Wesentlichen aus der druckabhängigen Ausdehnung der Membranschläuche resultiert. Im reversiblen Bereich ist diese nach dem Hooke'schen Gesetz proportional zum Druckunterschied zwischen Innen- und Außenseite der jeweiligen Membran und kann innerhalb einer referenzbasierten Messung über einen Proportionalitätsfaktor bei der Bestimmung des Wasser- bzw. Matrixpotentials berücksichtigt werden. Der Einfluss der elastischen Verformung lässt sich dabei in erster Näherung durch eine zu kalibrierende Konstante $\alpha$ berücksichtigen und das Wasser- bzw. Matrixpotential kann proportional zu $\Delta p(1 + \alpha)$ berechnet

werden. In einer Ausführungsform des Verfahrens ist daher vorgesehen dass eine druckabhängige elastische Verformung der Messkammern der Messzellen bei der Ermittlung des Wasser- und/oder Matrixpotentials $\overline{\psi}_{w/m}$ berücksichtigt wird.

## Bezugspunkt und Messbereich

Durch die Anordnung der Referenzzelle in der Referenzkammer kann ein definiertes Bezugspotential $\psi_0$ geschaffen werden, um das der Messbereich problemabhängig einstellbar ist. Durch dieses Bezugspotential wird, wie bei der Einführung des Funktionsprinzips beschrieben, der Nullpunkt des Messbereichs festgelegt, auf dem sich der Messbereich mit einer einstellbaren Weite erhebt.

Die Zusammensetzung der Referenzlösung und der Druck in der Referenzkammer bestimmen dieses Bezugspotential $\psi_0$. Durch eine geeignete Wahl dieses Bezugssystems können somit Beiträge von Komponenten im Wasserpotential auslegungsspezifisch im Messergebnis eliminiert werden. Eine osmotisch, beispielsweise an die Messumgebung angepasste Referenzlösung in der Referenzkammer kann genutzt werden, um das Matrixpotential isoliert vom osmotischen Potential zu bestimmen. Auch ein gegebenenfalls interessierendes Druckpotential kann in der Referenzkammer eingestellt oder durch die Umgebung aufgeprägt werden.

Die Festlegung des Messbereiches kann problemabhängig durch die Befüllung der Messkammern mit einer geeigneten osmotischen Lösung erfolgen.

In Ausführungsformen des Verfahrens ist daher vorgesehen, dass die hygroskopische(n) Substanz(en) und deren Konzentrationen innerhalb der osmotischen Lösungen in der Potentialzelle und in der Referenzzelle und/oder Referenzsubstanz(en) sowie deren Konzentrationen in der Referenzlösung in Abhängigkeit von Eigenschaften des Testkörpers und der Art der genutzten Membran gewählt werden oder gewählt sind.

Um auch einen Druck in der Referenzkammer einstellen zu können, ist in einer Ausführungsform vorgesehen, dass die Referenzkammer eine Vorrichtung zum Einstellen eines Drucks aufweist.

## Osmotische Lösung

Durch Lösung geeigneter hygroskopischer Substanzen in definierten Konzentrationen in Wasser können osmotische Lösungen mit vorgebbarem osmotischen Potential hergestellt werden. Neben Salzen (Elektrolyte) eignen sich hierzu beispielsweise wasserlösliche Polymere wie Polyethylenglycole, Polyacrylamide, Polysacharide u. a. wenn diese die Membran (diffusiv) nicht oder nur mit vernachlässigbarer Rate durchdringen können.

Polyethylenglycole (PEGs) sind linear verkettete Polyether, die in unterschiedlichen Kettenlängen (Wiederholeinheiten des Basismoleküls [-CH2-CH2-O-]) verfügbar sind. Sie sind biologisch abbaubar und besitzen breite Anwendung in Medizin und Kosmetik. Die Löslichkeit und die osmotische Wirksamkeit gelöster PEGs sinkt mit zunehmender Kettenlänge. Kurzkettige PEGs, z.B. PEG 400 oder PEG 800 (400 bzw. 800 Wiederholeinheiten) sind nahezu in jedem Verhältnis mit Wasser mischbar, womit bereits unterschiedliche Messbereiche ausgelegt werden können.

## Konstruktive Varianten des Tensiometers

Das Referenzsystem (insbesondere die Referenzkammer) kann beispielsweise durch einen geeigneten flexiblen Kunststoffschlauch, z.B. aus Polyurethane (PU), Polyvinylchlorid (PVC) etc. umhüllt werden. Ist die Druckstabilität des Referenzsystems gefordert, können dünnwandige Kapillaren aus Edelstahl oder geeigneten druckstabilen Kunststoffen genutzt werden. Die Schlauch-/Kapillarenden werden nach außen verschlossen bzw. enthalten Kapillaren und Ventile zur Befüllung der Referenzkammer. Um die Referenzkammer auf definiertem Druckniveau zu halten, kann ein äußerer Druck (Luftdruck, hydrostatischer Wasserdruck) über die Wandung der Referenzkammer aufgeprägt werden oder dem Kammerinneren über eine Kapillare zugeführt werden. Die Referenzzelle wird in geeigneter Weise in der Referenzkammer angeordnet, beispielsweise radialsymmetrisch durch diese hindurchgeführt.

Die Messzellen können zueinander baugleich ausgeführt sein. Insbesondere weisen die Messzellen dann gleiche Längen, Durchmesser und Wandstärken der Membranen auf und die nichtporösen, semipermeablen Membranen gleichen einander hinsichtlich Art und Herstellung.

Bei gleicher Temperatur im Bereich des Tensiometers, kann beispielsweise die Länge der Referenzzelle oder auch deren Volumen verkleinert werden. Gleiches gilt für das gesamte Referenzsystem, dass dann an geeigneter Position mit der nun vergleichsweise größeren Potentialzelle kombiniert werden kann. In einer Ausführungsform ist daher vorgesehen, dass das Referenzsystem im Vergleich zur Potentialzelle kleiner ausgeführt ist und an geeigneter Stelle im Tensiometer positioniert ist.

In einer Ausführungsform ist vorgesehen, dass das Tensiometer ein in die Potentialzelle oder je ein in Potential- und Referenzzelle eingebrachtes, sich entlang einer Längsachse der Messkammern der Messzellen erstreckendes Element aufweist, das in Bezug auf einen senkrecht zur Längsachse orientierten Querschnitt der Messkammern der Messzellen mittig angeordnet ist. Das Element kann beispielsweise zylinderförmig (Rundstab) oder auch mit abweichendem Querschnitt als Profilstab ausgebildet sein. Hierdurch wird der Volumenanteil der Messkammern, in dem sich die osmotischen Lösungen befinden, reduziert. Diese Volumenreduktion führt unter Berücksichtigung der unveränderten Geometrie der umhüllenden Membran zu einer schnelleren Einstellung der Drücke in den Messkammern, d.h. die Reaktionszeit des Tensiometers kann auf diesem Weg verkürzt werden. Das stabförmige Element weist einen Querschnitt auf, der es ermöglicht, diese so in die jeweilige Messkammer zu integrieren, dass zwischen Element und innerer Membranoberfläche ein mit der osmotischen Lösung befüllbares, radial möglichst gleichmäßig um das Element verteiltes Volumen verbleibt. Optimal ist eine radialsymmetrische Integration des Elements in die jeweilige Messkammer. Das Element wird dabei auf Länge und Durchmesser der jeweiligen Messkammer so abgestimmt, dass eine minimale Reaktionszeit des Tensiometers erreicht wird. Das Element ist flexibel oder starr ausgeführt und kann zur Lagedefinition über Stege verfügen, die spiralförmig, geradlinig oder ringförmig auf dessen Oberfläche angeordnet sind und in linien- oder punktförmigen Kontakten mit der Membranwand stehen. Alternativ kann ein flies- oder netzartiger Strumpf aus einem geeigneten (inkompressibel) Kunststoff zur Lagedefinition eingesetzt werden.

Wird in die Potentialkammer wie voranstehend beschrieben ein volumenreduzierendes Element integriert, ermöglicht dies eine weitere Bauform des Tensiometers, in der das Referenzsystem in die Messkammer der Potentialzelle integriert ist. In einer Ausführungsform ist daher vorgesehen, dass das Referenzsystem in die Potentialzelle über deren Länge integriert ist. In einer weiterbildenden Ausführungsform ist vorgesehen, dass das Referenzsystem zumindest teilweise das Element nach der voranstehend beschriebenen Ausführungsform ausbildet. Gleiche Längen beider Messzellen ermöglichen dann nach wie vor die Messung über Bereiche mit örtlich variierenden Temperaturen. Der durch die Integration des Referenzsystems verbleibende hohlzylinderförmige Raum in der Messkammer der Potentialzelle bewirkt die gewünschte Reduktion des mit der osmotischen Lösung befüllten Volumens und damit die Reduktion der Reaktionszeit des Tensiometers. Kammern, Membranen und die Wand des Referenzsystems können dabei so aufeinander abgestimmt angefertigt sein, dass die mit der osmotischen Lösung befüllten Volumina von Potential- und Referenzzelle übereinstimmen, oder dass eine reproduzierbare geometrische Relation von Wandstärken der Membranen, Membranflächen und Volumina der osmotischen Lösungen sicherstellt, dass die Einstellung des osmotischen Drucks, unabhängig von den verwendeten (ggf. unterschiedlichen) Membranmaterialien, in der Referenzzelle schneller oder gleich schnell im Vergleich zur Potentialzelle erfolgt.

## Mechanische Stabilisierung

Die Messkammern können in Kapillarrohre oder, insbesondere schlauchförmige, Geflechte integriert sein, die mit wasserpermeablen Aussparungen versehen sind und in die die jeweiligen Messzellen bündig eingepasst / eingepresst sind. In einer Ausführungsform ist daher vorgesehen, dass das Tensiometer mit permeablen Hohlräumen und/oder Poren versehene Kapillarrohre aufweist, in die die Membranen der Messzellen jeweils bündig eingepasst sind.

Alternativ können die Membranen der Messzellen zumindest teilweise als Füllung in die Aussparungen der Kapillarrohre oder Geflechte integriert sein. Daher ist in einer weiteren Ausführungsform vorgesehen, dass das Tensiometer mit Hohlräumen und/oder Poren versehene Kapillarrohre oder, insbesondere schlauchförmige, Geflechte aufweist, wobei die Membranen der Messzellen zumindest teilweise in die Hohlräume integriert sind.

Durch beide Varianten kann die elastische Ausdehnung der Messzellen reduziert werden, sodass die Volumina in den Messkammern auch bei hohen Drücken näherungsweise konstant gehalten werden können. Diese Art der Stabilisierung ermöglicht auch eine Reduktion der Wandstärke der Membranen und die Nutzung unterschiedlicher Polymere oder Ionomere bei näherungsweise gleichbleibendem Dehnungsverhalten. Zudem können die Membranen hierdurch vor äußeren mechanischen Einflüssen geschützt werden.

Die Kapillarrohre können beispielsweise geschlitzte Aussparungen besitzen, aus porösen gesinterten Metallen oder Keramiken bestehen oder auch aus Kunststoffen gefertigt sein. Geflechte können aus metallischen und/oder nichtmetallischen Fasern bestehen oder diese umfassen und die Membran mit definierter Maschenweite umhüllen und/oder einbetten.

## Integrierte Temperaturmessung

Das Tensiometer kann mit Temperatursensoren ausgerüstet sein, die in Abhängigkeit der Bauform in die Potentialzelle, die Referenzzelle oder beiden Messzellen integriert sind. In einer Ausführungsform ist daher vorgesehen, dass das Tensiometer mindestens einen Temperatursensor in oder an der Potentialzelle und/oder in oder an der Referenzzelle aufweist. Der mindestens eine Temperatursensor gestattet insbesondere, zumindest die Temperatur in der Potentialzelle oder der Referenzzelle zu erfassen. Hierdurch kann die Temperatur als wichtiger Parameter der Umgebungsbedingungen bereitgestellt werden. Die Messung der Temperaturdifferenz zwischen den Messzellen ermöglicht zudem temperaturabhängige Differenzdruckabhängigkeiten zwischen den Messzellen zu kompensieren bzw. entsprechende Unsicherheiten des Messergebnisses anzugeben. Die Kalibration von temperaturabhängigen Abhängigkeiten des Differenzdrucks $\Delta p$ erfolgt vorzugsweise experimentell in Abhängigkeit vorgegebener Temperaturen und Temperaturunterschiede.

Der Temperatursensor kann beispielsweise als drahtförmiger Widerstandssensor entlang der Längsachse der Messzelle integriert sein

Die zur Volumenreduktion genutzten Elemente können zumindest einen Teil dieser Temperatursensoren aufnehmen oder ausbilden. Daher ist in einer weiteren Ausführungsform vorgesehen, dass das Element zumindest einen Teil des Temperatursensors ausbildet.

## Integrierte Spannungsmessung

Das Tensiometer kann mit einer Messanordnung, umfassend eine Spannungsmesseinrichtung mit entsprechender Kontaktierung, ausgerüstet sein, die den elektrischen Potentialunterschied zwischen der Messkammer der Potentialzelle und deren unmittelbarer Umgebung als elektrische Spannung erfasst. Für eine, mit einer Ionomermembran aufgebauten Messzelle ist es dann möglich, Störungen bei der Messung des Matrixpotentials zu reduzieren bzw. zu eliminieren. Diese Spannung kann in Abhängigkeit des durch Salze bedingten osmotischen Potentialunterschiedes zwischen beiden Seiten der Membran, z.B. als Druckfunktion kalibriert, auf dem Tensiometerboard hinterlegt und zur Reduktion bzw. zum Eliminieren von variierenden osmotischen, dem Matrixpotential überlagerten Potentialdifferenzen genutzt werden. Zur Kalibration wird die elektrische Spannung zwischen der Innenseite der Membran (in der Messkammer) und deren Außenseite (nahe der Membranoberfläche) vorzugsweise experimentell in Abhängigkeit vorgegebener Ionenkonzentrationen bestimmt und der zugehörige osmotische Druck in der Potentialzelle bestimmt.

Das zur Volumenreduktion genutzte Element innerhalb der Messkammer der Potentialzelle kann einen der elektrischen Kontakte zur Messung der Spannung formen. Daher ist in einer weiteren Ausführungsform vorgesehen, dass das Element zumindest einen Teil eines elektrischen Kontaktes zur osmotischen Lösung in der Potentialzelle ausbildet, der mit der Spannungsmesseinrichtung verbunden ist. Dazu ist die Oberfläche des Elementes zumindest teilweise elektrisch leitend ausgebildet.

Das um die Potentialzelle angeordnete Kapillarrohr oder Geflecht kann (zumindest teilweise) elektrisch leitend ausgeführt werden und zumindest einen Teil des äußeren elektrischen Kontaktes für die Spannungsmessung bilden. Daher ist in einer Ausführungsform vorgesehen, dass zumindest das Kapillarrohr oder Geflecht, in dem die Potentialzelle angeordnet ist, zumindest teilweise elektrisch leitend ausgebildet ist und einen elektrischen Kontakt zur (feuchten) äußeren Membranoberfläche ausbildet, mit dem die Spannungsmesseinrichtung verbunden ist. Das Geflecht kann zu diesem Zweck beispielsweise metallische Fasern umfassen, durch die das äußere Potential abgegriffen werden kann.

## Befüllung der Messkammern

Die Messkammern können jeweils an beiden Enden Ein- bzw. Auslassventile besitzen. Daher ist in einer Ausführungsform vorgesehen, dass die Messzellen jeweils an beiden Enden ein Einlass-/Auslass-Ventil aufweisen. Hierdurch können die Messkammern bzw.

Messzellen befüllt, entleert, gespült und gereinigt werden. Insbesondere wird hierdurch der Austausch der osmotischen Lösung bei einem im Testkörper, z.B. im Boden verbauten, schlauchförmigen Tensiometer ermöglichet, ohne dieses auszubauen und damit, ohne die Messumgebung im Zuge dieser Wartungsarbeiten am Tensiometer zu stören. In diesem Zusammenhang kann eine Pumpvorrichtung zeitweilig oder permanent installiert und/oder genutzt werden, um die Messzellen zu entleeren, zu befüllen oder um Konzentrationsunterschiede der osmotischen Lösungen zwischen den Messzellen auszugleichen.

Zur Einstellung, Korrektur und Zustandsprüfung können die Konzentrationen bzw. entsprechende Konzentrationsunterschiede zwischen den Messzellen mit einer geeigneten Messeinrichtung zeitweilig oder permanent erfasst werden. Bei Verwendung hygroskopischer Salze kann beispielsweise die Leitfähigkeit zur Beurteilung der Konzentration gemessen werden. In einer Ausführungsform ist daher vorgesehen, dass das Tensiometer eine Konzentrationsmesseinrichtung aufweist, die eingerichtet ist zum Erfassen zumindest einer Konzentrationsdifferenz einer hygroskopischen Substanz innerhalb der osmotischen Lösungen in den Messzellen.

Die hygroskopische(n) Substanz(en) in den Messkammern und/oder die Referenzsubstanz(en) können problemabhängig in Abhängigkeit von Eigenschaften des Testkörpers und der verwendeten Membranart gewählt werden oder voreingestellt sein. Hierdurch kann der Messbereich in Lage und Größe vorgegeben und während des Betriebs angepasst werden. Der Messbereich lässt sich beispielsweise durch die Konzentration(en) und Art(en) der hygroskopischen Substanz(en) einstellen, da sich hierdurch das osmotische Potential der Lösungen gezielt beeinflussen lässt.

Ferner kann die Referenzlösung in einer zum Testkörper osmotisch gleichwertigen Zusammensetzung verwendet werden. Wird als Membranmaterial eine für Ionen impermeable Membran verwendet, so kann das osmotische Potential dadurch messtechnisch eliminiert werden, wodurch die zwischen den Messkammern erfasste Druckdifferenz näherungsweise das Matrixpotential bestimmt. In einer Ausführungsform des Verfahrens ist daher vorgesehen, dass als osmotische Referenzlösung eine wässrige Lösung mit einer zum Testkörper osmotisch näherungsweise gleichwertigen Zusammensetzung verwendet wird.

### Datenverarbeitung

Der am Drucksensor bestimmte Druckwert wird durch eine zweckdienliche elektronische Schaltung zur Datenverarbeitung als geeignetes Signal, beispielsweise als Datenwert oder Datenpaket aufbereitet, in die gewünschte Darstellungsform für das Wasser- oder Matrixpotential konvertiert, geeignet korrigiert und ggf. gemeinsam mit weiteren Parametern (Temperaturen, Systemparameter) gespeichert, ausgegeben, dargestellt und/oder über einen datenbusfähigen Ausgang bereitgestellt. Die hierfür nötigen elektronischen Komponenten und Programmcodes sind in einem Tensiometerboard implementiert.

### Einbauabhängige Varianten

Wegen der linienförmigen Geometrien von Potentialzelle und Referenzsystem, welche flexibel ausgebildet werden können, kann ein solches Tensiometers als gemeinsamer Strang durch den Bodenkörper, das Substrat oder Medium geführt werden.

Soll das Tensiometer in den Testkörper einstechbar ausgebildet werden, so kann das Referenzsystem äußerlich mechanisch versteift werden, z.B. mit einem perforierten Hohlprofil. Gleiches gilt für diesen Fall für die dem Testkörper ausgesetzte Potentialzelle.

Durch seinen linienförmigen, flexibel oder steif auslegbaren Aufbau kann das Tensiometer in Art und Länge sowohl an eine konkrete Fragestellung, z.B. an die Heterogenität des Standortes, die Größe des Pedons, die Wurzeldichteverteilung usw. angepasst werden als auch an die Art des Einbaus in den Testkörper, z.B. durch Verlegen in geeignet vorbereitete, z.B. durch Schlitzen oder Vorbohren zugängliche Bereiche des Testkörpers oder durch Einstechen in diesen (ggf. wiederum nach Vorbohren um eine lokale Materialverdichtung zu vermeiden).

### Anwendungsbereich

Neben dem Einsatz in Böden kann das Tensiometer auch in anderen Substraten oder Medien zur Bestimmung des dort herrschenden Wasserzustands eingesetzt werden, so beispielsweise auch zur präzisen Bestimmung der relativen Luftfeuchtigkeit nahe 100 %, d.h. dort, wo herkömmliche Feuchtesensoren generell ungenau werden. Die Aufrechterhaltung einer möglichst hohen Luftfeuchtigkeit bei gleichzeitiger Vermeidung der Kondenswasserbildung ist beispielsweise für die Lagerung von Obst nötig.

### Zusammenfassung

Die Erfindung betrifft einen linienförmigen Wasserpotentialsensor (Tensiometer) wählbarer Länge mit justierbarem Messbereich zum Einsatz in trockenen und feuchten, heterogen zusammengesetzten Böden, Substraten oder Medien und ein referenzbasiertes Verfahren zur repräsentativen Bestimmung des Wasserpotentials, z.B. entlang eines Horizontes in diesem Testkörper durch die räumlich entlang des Tensiometers gemittelte Erfassung der lokal vorhandenen Wasserpotentiale.

Das Tensiometer und das beschriebene Verfahren ermöglichen die Bestimmung des über den Testkörper, insbesondere einen pedonskaligen Bodenkörper, gemittelten Wasser-oder Matrixpotentials. Darüber hinaus kann über eine geeignete Wahl der hygroskopischen Substanz(en) und deren Konzentration in der genutzten osmotischen Lösung und/oder der Referenzsubstanz(en) und deren Konzentration in der genutzten Referenzlösung ein Messbereich in Lage und Größe eingestellt werden, der optimal an den Dynamikbereich des Wasserpotentials im Testkörpers angepasst ist.

Auf diese Weise wird ein, über einen definierbaren internen Standard referenziertes, diffusionsbasiertes Tensiometer geschaffen, das in seinem räumlichen Mittelungsverhalten sowie in Größe und Lage des Messbereichs an das jeweilige Messproblem anpassbar ist.

Nachfolgend wird die Erfindung anhand bevorzugter Ausführungsbeispiele unter Bezugnahme auf die Figuren näher erläutert. Hierbei zeigen:

Fig. 1      eine schematische Darstellung einer Ausführungsform des Tensiometers;

Fig. 2      eine schematische Darstellung eines Ausschnitts des Tensiometers zur Verdeutlichung einer weiteren Ausführungsform des Tensiometers;

Fig. 3      eine schematische Darstellung eines Ausschnitts des Tensiometers zur Verdeutlichung einer weiteren Ausführungsform des Tensiometers;

Fig. 4      eine schematische Darstellung einer weiteren Ausführungsform des Tensiometers;

Fig. 5      ein schematisches Ablaufdiagramm einer Ausführungsform des Verfahrens zur Bestimmung eines räumlich gemittelten Wasserpotentials in einem Testkörper.

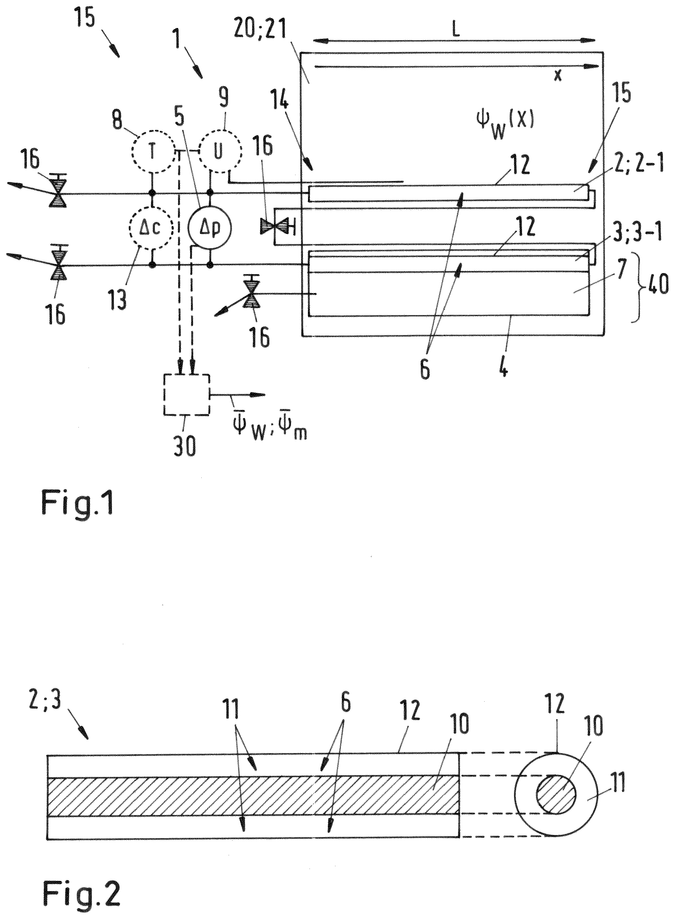

In Fig. 1 ist eine schematische Darstellung einer Ausführungsform des Tensiometers 1 gezeigt. Das Tensiometer 1 umfasst eine Potentialzelle 2 und ein Referenzsystem 40 mit einer Referenzzelle 3 und mit einer Referenzkammer 4 und eine Druckmesseinrichtung 5.

Die Potentialzelle 2 umfasst eine mit einer schlauchförmigen semipermeablen Membran 12 ummantelte verschließbare Messkammer 2-1. Die Membran 12 ist ein wasserpermeables Polymer (beispielsweise Silikon) oder ein Ionomere (beispielsweise Nafion) oder ein in der allgemeinen Beschreibung offenbartes oder funktional gleichwertiges Material.

Die Referenzzelle 3 umfasst eine mit einer schlauchförmigen semipermeablen Membran 12 ummantelte verschließbare Messkammer 3-1. Die Referenzzelle 3 ist insbesondere baugleich zur Potentialzelle 2 ausgebildet. Durchmesser, Länge und Wandstärke der Membranen 12 beider Messkammern 2-1, 3-1 sowie das Membranmaterial gleichen einander. Die Referenzzelle 3 ist in die Referenzkammer 4 integriert.

Alternativ kann grundsätzlich auch vorgesehen sein, dass das Referenzsystem 40 im Vergleich zur Potentialzelle 2 kleiner ausgeführt ist und an geeigneter Stelle im Tensiometer 1 positioniert ist.

Die Potentialzelle 2 und die Referenzzelle 3 sind mit einer osmotischen Lösung 6 befüllt, wobei die Lösung 6 in beiden Messkammern 2-1, 3-1 die gleiche ist. Die osmotische Lösung 6 kann mit Salzen und/oder wasserlöslichen Polymeren, z.B. PEGs, mit geeigneter Kettenlänge hergestellt werden.

Die in die Referenzkammer 4 integrierte Referenzzelle 3 ist von der Referenzlösung 7 umgeben. Diese Referenzlösung 7 definiert mit dem Bezugspotential den Bezugspunkt für die Messung des Wasserpotentials. Die Referenzlösung 7 kann beispielsweise Wasser sein. Die Referenzkammer 4 wird beispielsweise durch einen flüssigkeitsdichten Schlauch oder Sack aus einem geeigneten Kunststoff, wie Polyurethan (PU) oder Polyvinylchlorid (PVC) etc. gebildet.

Die Druckmesseinrichtung 5 ermöglicht, zumindest die Druckdifferenz $\Delta p$ zwischen der Potentialzelle 2 und der Referenzzelle 3 zu erfassen, beispielsweise mittels eines Differenzdrucksensors.

Die Potentialzelle 2 und das Referenzsystem 40 (Referenzzelle 3 und Referenzkammer 4) werden den gleichen Umgebungsbedingungen ausgesetzt. Insbesondere soll in den Messzellen 2, 3 die gleiche Temperatur herrschen. Mindestens ein Temperatursensor 8 kann zur Messung einer Temperatur T vorgesehen sein, mit dem zumindest eine Temperatur der Potentialzelle 2 erfasst wird.

Die Potentialzelle 2 und das Referenzsystem 40 (Referenzzelle 3 und Referenzkammer 4) werden in einem Testkörper 20, insbesondere in einem Boden 21, in dem das Wasserpotential oder das Matrixpotential bestimmt werden soll, angeordnet. Hierbei ist insbesondere vorgesehen, dass die Messkammern 2, 3 eng benachbart, zum Beispiel gemeinsam als Strang, durch den Testkörper 20, 21 geführt werden. Vereinfacht ist dies schematisch in der Fig. 1 zu sehen, wo sich die Messzellen 2, 3 und die Referenzkammer 4 im Testkörper 20 entlang einer symbolischen Richtung x erstrecken. Bei Anwendung des Tensiometers 1 im Boden können die Messzellen 2, 3 und die Referenzkammer 4 entlang beliebiger Raumrichtungen verlaufen, sodass eine Lage des Tensiometers 1 im Testkörper 20 in Abhängigkeit von der jeweiligen Fragestellung festgelegt werden kann und damit der Bereich, über den das gemittelte Wasser- oder Matrixpotential erfasst werden soll. Die Messzellen 2, 3 und die Referenzkammer 4 können hierbei eine Länge L im Bereich von einigen Zentimetern bis hin zu vielen Metern aufweisen.

Mittels des Tensiometers 1 kann die Bestimmung des Wasser- oder Matrixpotentials $\overline{\psi}_{w/m}$ im Testkörper 20 durchgeführt werden, wobei die Druckdifferenz $\Delta p$ zwischen der Potentialzelle 2 und der Referenzzelle 3 mittels der Druckmesseinrichtung 5 erfasst wird. Ausgehend von dieser Druckdifferenz $\Delta p$ wird das Wasser-

oder Matrixpotentials im Testkörper 20 - einem Boden 21 oder anderem Substrat - gemessen und bereitgestellt. Dies erfolgt insbesondere, nachdem sich Gleichgewichte der Wasseraktivitäten zwischen den osmotischen Lösungen 6 in den Messzellen 2, 3 einerseits und den Wässern in deren Umgebung (Testkörper 20, Referenzkammer 4) andererseits eingestellt haben.

Zur Bestimmung des Wasser- oder Matrixpotentials besitzt das Tensiometer 1 ein Tensiometerboard 30, das elektronische Komponenten, beispielsweise einen programmierten Mikrocontroller und einen Speicher, umfasst. Das Tensiometerboard 30 ist insbesondere Teil des Tensiometers 1. Das Tensiometerboard 30 wertet insbesondere das Signal der Druckmesseinrichtung 5 aus und erzeugt hieraus einen Messwert für das über die Länge L gemittelte Wasser- bzw. Matrixpotential $\overline{\psi}_{w/m}$.

Es kann vorgesehen sein, dass das Tensiometer 1 eine Spannungsmesseinrichtung 9 aufweist, die eine elektrische Spannung $U$ zwischen einer Innenseite und einer Außenseite der Potentialzelle 2 erfasst. Es ist dann vorgesehen, dass mittels der Spannungsmesseinrichtung 9 die Spannung $U$ zur Korrektur elektrolytkonzentrationsabhängig vorhandener osmotischer Potentialdifferenzen zwischen der Potentialzelle 2 und einem Außenraum ermittelt wird und basierend auf einer Kalibration des assoziierenden Drucks in der Potentialzelle 2 vom ermittelten Differenzdruck $\Delta p$ reduziert bzw. eliminiert wird, um das über die Länge L gemittelte Matrixpotential $\overline{\psi}_m$ für den Wasserzustand im Testkörper 20, insbesondere im Boden 21, zu berechnen.

Es kann vorgesehen sein, dass das Tensiometer 1 ein in die Potentialzelle 2 und in die Referenzzelle 3 jeweils eingebrachtes stabförmiges Element 10 aufweist, dass sich entlang der Messkammern 2-1 und 3-1 erstreckt und mittig darin angeordnet ist. Diese Ausführungsform des Tensiometers 1 ist als vergrößerter Ausschnitt schematisch in der Fig. 2 verdeutlicht. Das Element 10 kann beispielsweise als Profil- oder Rundstab ausgebildet sein und dient der Verringerung des Messkammervolumens, in welchem sich die osmotische Lösung befindet. In der gezeigten Ausführungsform reduziert sich das Messkammervolumen auf einen hohlzylinderförmigen Ringraum 11 zwischen dem Element 10 und der inneren Oberfläche der Membran 12. Hierzu ist das Element 10 in Bezug auf den Querschnitt der jeweiligen Messzellen 2 und 3 mittig angeordnet. Hierzu können auch Positioniermittel genutzt werden, die die mittige Anordnung des Elementes 10 aufrechterhalten, wie in der allgemeinen Beschreibung offenbart. Durch die Verringerung des Messkammervolumens reduziert sich insbesondere eine Einstellzeit für den Druck innerhalb der Messzellen 2, 3, sodass das Wasserpotential mit zeitlich größerer Auflösung bestimmt werden kann. Das Element 10 ist insbesondere biegsam, sodass die Flexibilität der schlauchförmigen Messzellen 2, 3 nicht eingeschränkt wird.

Darüber hinaus kann vorgesehen sein, dass das Element 10 zumindest einen Teil eines Temperatursensors 8 und/oder eines elektrischen Kontaktes zur osmotischen Lösung für die Spannungsmesseinrichtung 9 umfasst. Hierzu ist in dem Element 10, beispielsweise in dem Profil- oder Rundstab, beispielsweise ein temperaturabhängiger Widerstand angeordnet und/oder der Profil- oder Rundstab bildet äußerlich zumindest teilweise eine elektrisch leitfähige Elektrode aus, die in Kontakt mit einer Spannungsmesseinrichtung 9 steht.

Es kann weiterbildend auch vorgesehen sein, dass das Referenzsystem 40 in die Potentialzelle 2 über deren Länge integriert ist. Es kann dann insbesondere vorgesehen sein, dass das Referenzsystem 40 zumindest teilweise das Element 10 ausbildet.

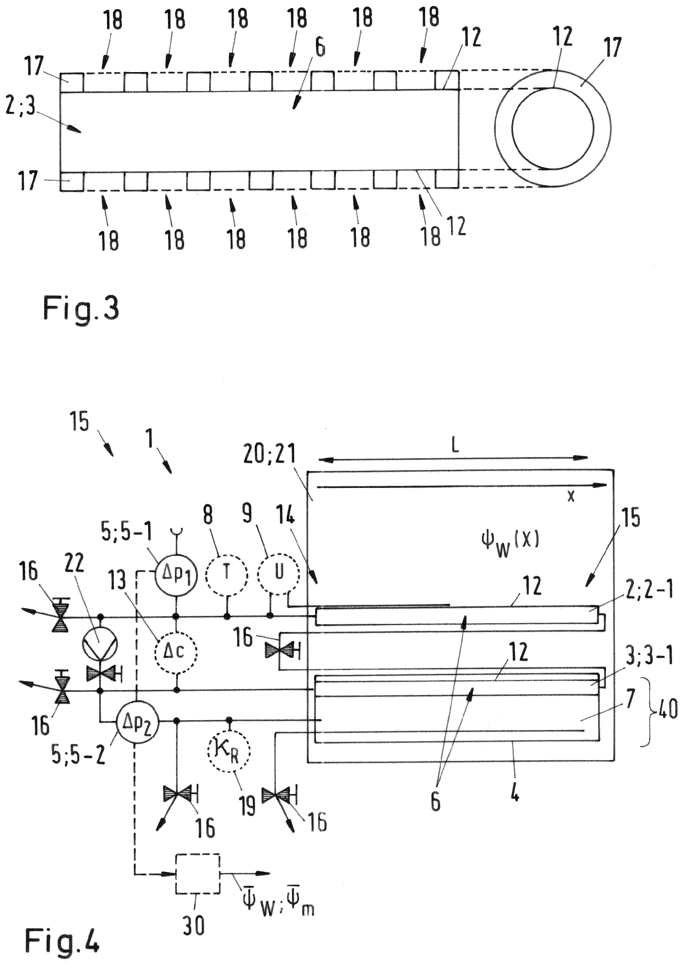

Die Fig. 3 zeigt eine schematische Darstellung eines Ausschnitts einer weiteren Ausführungsform des Tensiometers, bei der vorgesehen ist, dass das Tensiometer Kapillarrohre 17 mit wasserpermeablen perforierten, geschlitzten oder porösen Hohlräumen 18 besitzt. Anstatt eines steifen Kapillarrohres 17 können auch, insbesondere schlauchförmige, Geflechte vorgesehen sein. Diese Geflechte können aus metallischen und/oder nichtmetallischen Fasern bestehen oder solche umfassen. Die Membranen 12 der Messzellen 2 bzw. 3 sind entweder bündig in die Kapillarrohre 17 integriert, füllen die Hohlräume der Wandung der Kapillarrohre 17 oder werden bündig/kraftschlüssig von einem Geflecht mit definierter Maschenweite umhüllt und/oder in dieses eingebettet. Das Wasser der jeweiligen Umgebung steht über die Hohlräume 18 mit den Membranen 12 der Messzellen 2 und 3 in Kontakt. Hierdurch wird ein Schutz gegen mechanische Einflüsse erreicht und die durch den wechselnden Druck in den Messzellen 2,3 hervorgerufene, variierende elastische Ausdehnung der Membranen 12 begrenzt. Die Kapillarrohre/Geflechte 17 können fallweise aus einem Kunststoff, Metall oder einer Keramik bestehen. Sie können auch aus dem Material einer geeigneten semipermeablen Membran bestehen, die für Wasser durchlässig ist.

Darüber hinaus kann dabei vorgesehen sein, dass die druckabhängige elastische Verformung der Messzellen 2, 3 in der voranstehend beschriebenen Weise kalibriert und bei der Bestimmung des Wasser- bzw. Matrixpotentials berücksichtigt wird.

Ferner kann vorgesehen sein, dass zumindest das Kapillarrohr/Geflecht 17, in dem die Potentialzelle 2 angeordnet ist, zumindest teilweise elektrisch leitend ist und zumindest teilweise elektrisch leitend ausgebildet ist und einen elektrischen Kontakt zur (feuchten) äußeren Membranoberfläche ausbildet, mit dem die Spannungsmesseinrichtung 9 verbunden ist. Das Geflecht kann hierzu beispielsweise eine oder mehrere metallische Fasern umfassen, mit der/denen ein äußeres Potential abgegriffen werden kann.

Es kann vorgesehen sein, dass das Tensiometer 1 eine Konzentrationsmesseinrichtung 13 (Fig. 1) aufweist, die die Konzentrationsdifferenz $\Delta c$ der hygroskopischen Substanz zwischen den Messzellen 2, 3 unter Nutzung des Tensiometerboards 30 erfasst. Dies ermöglicht es, nach dem Befüllen oder Wechseln der osmotischen Lösung 6 zu prüfen, ob in den Messzellen 2, 3 hinreichend ähnliche Konzentrationen vorliegen.

Es kann vorgesehen sein, dass die Messzellen 2 und 3 jeweils an beiden Enden 14, 15 Ein- und Auslassventile 16 besitzen (Fig. 1). Hierdurch kann ein Befüllen und Entleeren der Messzellen 2, 3 erleichtert werden. Es kann ferner vorgesehen sein, dass die Referenzkammer 4 ein Ein- und Auslassventil 16 aufweist. In der in der Fig. 1 gezeigten Ausführungsform können die Messzellen 2 und 3 über das Ein- und Auslassventil 16 miteinander verbunden werden. Dies ermöglicht den Konzentrationsausgleich der osmotischen Lösungen 6 zwischen den Messzellen 2, 3 und erleichtert deren gleichmäßige Befüllung.

In der Fig. 4 ist eine schematische Darstellung einer weiteren Ausführungsform des Tensiometers 1 gezeigt. Die Ausführungsform ist grundsätzlich wie die in der Fig. 1 gezeigte Ausführungsform ausgestaltet, gleiche Bezugzeichen bezeichnen gleiche Merkmale und Begriffe. Bei dieser Ausführungsform ist vorgesehen, dass die Druckmesseinrichtung 5 einen ersten Differenzdrucksensor 5-1 und einen zweiten Differenzdrucksensor 5-2 aufweist. Der erste Differenzdrucksensor 5-1 erfasst die Druckdifferenz $\Delta p_1$ zwischen der Potentialzelle 2 und der Atmosphäre. Der zweite Differenzdrucksensor 5-2 erfasst eine Druckdifferenz $\Delta p_2$ zwischen der Referenzzelle 3 und der Atmosphäre. Aus der Differenz der beiden Differenzdrücke $\Delta p_1$, $\Delta p_2$ lässt sich der Differenzdruck $\Delta p$ zwischen den beiden Messzellen 2, 3 bestimmen, woraus sich das über die Länge L gemittelte Wasserpotential $\overline{\psi_w}$ oder Matrixpotential $\overline{\psi_m}$ in der voranstehend beschriebenen Weise bestimmen lassen.

Ferner kann vorgesehen sein, dass das Tensiometer 1 eine Pumpvorrichtung 22 aufweist. Diese kann temporär installiert und/oder genutzt werden, um Konzentrationsunterschiede zwischen beiden Messkammern 2-1, 3-1 auszugleichen, beispielsweise, nach einem Austausch der in den Messkammern 2-1, 3-1 befindlichen osmotischen Lösung 6.

Ferner kann vorgesehen sein, dass das Tensiometer 1 einen Leitfähigkeitssensor 19 aufweist, um die Leitfähigkeit $\kappa_R$ der Referenzlösung 7 zu bestimmen. Dies ermöglicht es, eine Ionenstärke bzw. Konzentration der Referenzlösung 7 in der Referenzkammer 4 zu prüfen und einzustellen.

In einem vorbereitenden Schritt kann vorgesehen sein, dass die hygroskopische(n) Substanz(en) in den Messkammern und/oder die Referenzsubstanz(en) in der Referenzkammer 4 in Abhängigkeit von Eigenschaften des Testkörpers gewählt werden oder gewählt sind. Gleiches gilt für die Konzentrationen der jeweiligen osmotischen Lösungen. Die Auswahl der hygroskopischen Substanz(en) und/oder der Referenzsubstanz(en) kann hierbei auf Grundlage von empirisch, in Versuchsreihen gewonnenen Erkenntnissen basieren. Es kann dabei beispielsweise vorgesehen sein, dass mittels geeigneter Referenzsubstanz(en) eine wässrige Lösung mit einer zum Testkörper osmotisch gleichwertigen Zusammensetzung hergestellt wird. Insbesondere kann hierdurch das Matrixpotential direkt über die erfasste Druckdifferenz bestimmt werden, wenn ein geeignetes Polymer als Membran verwendet wird.

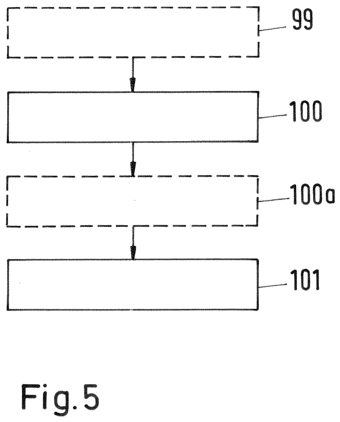

In der Fig. 5 ist ein schematisches Ablaufdiagramm einer Ausführungsform des Verfahren zur Bestimmung eines räumlich gemittelten Wasserpotentials in einem Testkörper gezeigt.

In einer Maßnahme 100 wird mittels eines Tensiometers nach einer der voranstehend beschriebenen Ausführungsformen zumindest eine Druckdifferenz zwischen den Messkammern der Potentialzelle und der Referenzzelle erfasst.

In einer Maßnahme 101 wird ausgehend von der erfassten Druckdifferenz in Abhängigkeit von der verwendeten semipermeablen Membran und des Bezugspotentials das über die Länge der Potentialzelle gemittelte Wasser- und/oder Matrixpotential im Testkörper bestimmt und als Messwert bereitgestellt. Dies erfolgt insbesondere mittels des Tensiometerboards.

Es kann in einer Maßnahme 100a vorgesehen sein, dass das Matrixpotential im Testkörper in mit Ionomermembranen ausgestatteten Messzellen ermittelt wird, indem die elektrische Spannung zwischen der Innenseite und der Außenseite der Membran der Potentialzelle mittels der Spannungsmesseinrichtung erfasst wird, die nach Kalibration ermöglicht, einen Druck zu ermitteln, der durch elektrolytkonzentrationsabhängige osmotische Potentialunterschiede beidseitig der Membran bedingt ist, und diesen aus der gemessenen Druckdifferenz zu eliminieren und so das Matrixpotential zu bestimmen. Dies erfolgt ebenfalls mittels des Tensiometerboards. Das ermittelte Matrixpotential wird ebenfalls bereitgestellt.

In einer vorbereitenden Maßnahme 99 kann vorgesehen sein, dass die hygroskopische(n) Substanz(en) und deren Konzentrationen innerhalb der osmotischen Lösungen in der Potentialzelle und in der Referenzzelle sowie deren Konzentrationen in der Referenzlösung in Abhängigkeit von Eigenschaften des Testkörpers und der Art der genutzten Membran gewählt werden oder gewählt sind. Die Auswahl der hygroskopischen Substanz(en) kann hierbei beispielsweise auf Grundlage von empirisch in Versuchsreihen gewonnenen Erkenntnissen basieren.

Es kann in Maßnahme 99 beispielsweise vorgesehen sein, dass als Referenzlösung eine wässrige Lösung mit einer zum Testkörper osmotisch näherungsweise gleichwertigen Zusammensetzung verwendet wird. Insbesondere kann hierdurch das Matrixpotential direkt über die erfasste Druckdifferenz bestimmt werden, da das osmotische Potential und das durch die Referenzsubstanz vorgegebene Referenzpotential den gleichen Wert aufweisen.

Es kann in Maßnahme 101 vorgesehen sein, dass eine druckabhängige elastische Verformung der Messkammern der Messzellen bei der Ermittlung des Wasser- und/oder Matrixpotentials berücksichtigt wird.

Das in dieser Offenbarung beschriebene Tensiometer und das beschriebene Verfahren ermöglichen insbesondere das Bestimmen eines räumlich über einen Testkörper gemittelten Wasserpotentials. Ferner lässt sich mittels der beschriebenen Ausführungsformen auch das Matrixpotential bestimmen. Darüber hinaus kann über eine geeignete Wahl der osmotischen Lösung und/oder der Referenzlösung ein Messbereich eingestellt werden, sodass das Tensiometer an Eigenschaften des Testkörpers angepasst werden kann.

Bezugszeichenliste

| | |
|---|---|
| 1 | Tensiometer |
| 2 | Potentialzelle |
| 2-1 | Messkammer |
| 3 | Referenzzelle |
| 3-1 | Messkammer |
| 4 | Referenzkammer |
| 5 | Druckmesseinrichtung |
| 5-1 | Differenzdrucksensor |
| 5-2 | Differenzdrucksensor |
| 6 | osmotische Lösung |
| 7 | osmotische Referenzlösung |
| 8 | Temperatursensor |
| 9 | Spannungsmesseinrichtung |
| 10 | Element |
| 11 | hohlzylinderförmiger Ringraum |
| 12 | Membran |
| 13 | Konzentrationsmesseinrichtung |
| 14 | Ende |
| 15 | Ende |
| 16 | Einlass-/Auslassventil |
| 17 | Kapillarrohr (oder Geflecht) |
| 18 | Hohlraum |
| 19 | Leitfähigkeitssensor |
| 20 | Testkörper |
| 21 | Boden |
| 22 | Pumpvorrichtung |
| 30 | Tensiometerboard |
| L | Länge der Messkammern |
| T | Temperatur |
| U | elektrische Spannung |
| x | Einbaurichtung des Tensiometers |
| $\Delta c$ | Konzentrationsdifferenz |
| $\kappa_R$ | Leitfähigkeit |
| $\Delta p$ | Druckdifferenz |
| $\Delta p_1$ | Druckdifferenz |
| $\Delta p_2$ | Druckdifferenz |
| $\overline{\psi}_m$ | gemitteltes Matrixpotential |
| $\overline{\psi}_w$ | gemitteltes Wasserpotential |

**Patentansprüche**

1. Tensiometer (1), umfassend:

   - eine als Potentialzelle (2) bezeichnete Messzelle wählbarer Länge, die eine mit einer schlauchförmigen semipermeablen Membran (12) ummantelte verschließbare Messkammer (2-1) umfasst und in einen Testkörper (20) eingebaut werden kann;
   - ein Referenzsystem (40) umfassend eine Referenzkammer (4) und eine als Referenzzelle (3) bezeichnete Messzelle wählbarer Länge, die eine mit einer schlauchförmigen semipermeablen Membran (12) ummantelte verschließbare Messkammer (3-1) umfasst, wobei die Referenzzelle (3) in die Referenzkammer (4) des Referenzsystems (40) integriert ist, und wobei die Referenzkammer (4) eine osmotische Referenzlösung (7) enthält oder mit einer solchen befüllbar ist, wobei die Potentialzelle (2) und die Referenzzelle (3) mit einer osmotischen Lösung (6) befüllt oder befüllbar sind; und
   - eine Druckmesseinrichtung (5), die dazu eingerichtet ist, zumindest eine Druckdifferenz ($\Delta p$) zwischen den Messkammern (2-1,3-1) der Potentialzelle (2) und der Referenzzelle (3) zu messen.

2. Tensiometer (1) nach Anspruch 1, **dadurch gekennzeichnet, dass** die Referenzzelle (3) baugleich zur Potentialzelle (2) ausgeführt ist.

3. Tensiometer (1) nach Anspruch 1, **dadurch gekennzeichnet, dass** das Referenzsystem (40) im Vergleich zur Potentialzelle (2) kleiner ausgeführt ist und an geeigneter Stelle im Tensiometer (1) positioniert ist.

4. Tensiometer (1) nach einem der vorangegangenen Ansprüche, **dadurch gekennzeichnet, dass** die Referenzkammer (4) eine Vorrichtung zum Einstellen eines Drucks aufweist.

5. Tensiometer (1) nach einem der vorangegangenen Ansprüche, **gekennzeichnet durch** eine Spannungsmesseinrichtung (9) zur Messung einer elektrischen Spannung (U) zwischen der Innenseite und der Außenseite der Membran (12) der Potentialzelle (2).

6. Tensiometer (1) nach einem der vorangegangenen Ansprüche, **gekennzeichnet durch** ein in die Potentialzelle (2) oder je ein in Potential- und Referenzzelle (2, 3) eingebrachtes, sich entlang einer Längsachse der Messkammern (2-1,3-1) der Messzellen (2, 3) erstreckendes Element (10), das in Bezug auf einen senkrecht zur Längsachse orientierten Querschnitt der Messkammern (2-1,3-1) der Messzellen (2, 3) mittig angeordnet ist.

7. Tensiometer (1) nach einem der vorangegangenen Ansprüche, **dadurch gekennzeichnet, dass** das Referenzsystem (40) in die Potentialzelle (2) über deren Länge integriert ist.

8. Tensiometer (1) nach Anspruch 7, **dadurch gekennzeichnet, dass** das Referenzsystem (40) zumindest teilweise das Element (10) nach Anspruch 6 ausbildet.

9. Tensiometer (1) nach einem der vorangegangenen Ansprüche, **gekennzeichnet durch** mit permeablen Hohlräumen und/oder Poren (18) versehene Kapillarrohre (17) oder Geflechte, in die die Membranen (12) der Messzellen (2, 3) jeweils bündig eingepasst oder in die Hohlräume zumindest teilweise integriert sind.

10. Tensiometer (1) nach einem der vorangegangenen Ansprüche, **gekennzeichnet durch** mindestens einen Temperatursensor (8) in oder an der Potentialzelle (2) und/oder in oder an der Referenzzelle (3).

11. Tensiometer (1) nach einem der Ansprüche 6 bis 10, **dadurch gekennzeichnet, dass** das Element (10) zumindest einen Teil des Temperatursensors (8) und/oder eines elektrischen Kontaktes zur osmotischen Lösung in der Potentialzelle (2) ausbildet, der mit der Spannungsmesseinrichtung (9) verbunden ist.

12. Tensiometer (1) nach einem der Ansprüche 9 bis 11, **dadurch gekennzeichnet, dass** zumindest das Kapillarrohr (17) oder das Geflecht, in dem die Potentialzelle (2) angeordnet ist, zumindest teilweise elektrisch leitend ausgebildet ist und einen elektrischen Kontakt zur (feuchten) äußeren Membranoberfläche ausbildet, mit dem die Spannungsmesseinrichtung (9) verbunden ist.

13. Tensiometer (1) nach einem der vorangegangenen Ansprüche, **dadurch gekennzeichnet, dass** die Messzellen (2,3) jeweils an beiden Enden (14,15) ein Einlass-/Auslass-Ventil (16) aufweisen.

14. Tensiometer (1) nach einem der vorangegangenen Ansprüche, **gekennzeichnet durch** eine Konzentrationsmesseinrichtung (13), die eingerichtet ist zum Erfassen zumindest einer Konzentrationsdifferenz ($\Delta c$) einer hygroskopischen Substanz innerhalb der osmotischen Lösungen (6) in den Messzellen (2, 3).

15. Verfahren zur Bestimmung eines räumlich gemittelten Wasserpotentials ($\overline{\psi}_W$) in einem Testkörper (20), wobei mittels des Tensiometers (1) nach einem der Ansprüche 1 bis 15 zumindest die Druckdifferenz ($\Delta p$) zwischen den Messkammern (2-1,3-1) der Potentialzelle (2) und der Referenzzelle (3) erfasst wird, die aufgrund ihrer Anordnung innerhalb der Referenzkammer (4) des Referenzsystems (40) den Bezug zu einem wählbaren Bezugspotential gestattet, wobei ausgehend von der erfassten Druckdifferenz ($\Delta p$) in Abhängigkeit von der verwendeten semipermeablen Membran und des Bezugspotentials das über die Länge der Potentialzelle (2) gemittelte Wasser- und/oder Matrixpotential ($\overline{\psi}_{w/m}$) im Testkörper (20) bestimmt und als Messwert bereitgestellt wird.

**Claims**

1. A tensiometer (1), comprising:

   - a measuring cell of selectable length, referred to as a potential cell (2), which comprises a closable measuring chamber (2-1) sheathed in a tubular semi-permeable membrane (12) and can be installed in a test piece (20);
   - a reference system (40) comprising a reference chamber (4) and a measuring cell of selectable length, referred to as a reference cell (3), which comprises a closable measuring chamber (3-1) sheathed in a tubular semi-permeable membrane (12), wherein the reference cell (3) is integrated in the reference chamber (4) of the reference system (40), and wherein the reference chamber (4) contains an osmotic reference solution (7) or can be filled with such a solution, wherein the potential cell (2) and the reference cell (3) are or can be filled with an osmotic solution (6); and
   - a pressure-measuring apparatus (5), which is configured to measure at least one pressure difference ($\Delta p$) between the measuring chambers (2-1, 3-1) of the potential cell (2) and the reference cell (3).

2. The tensiometer (1) according to claim 1, **characterized in that** the reference cell (3) is structurally identical to the potential cell (2).

3. The tensiometer (1) according to claim 1, **characterized in that** the reference system (40) is smaller than the potential cell (2) and is positioned at a suitable point in the tensiometer (1).

4. The tensiometer (1) according to any one of the preceding claims, **characterized in that** the reference chamber (4) comprises a device for setting a pressure.

5. The tensiometer (1) according to any one of the preceding claims, **characterized by** a voltage-measuring apparatus (9) for measuring an electrical voltage (U) between the inner face and the outer face of the membrane (12) of the potential cell (2).

6. The tensiometer (1) according to any one of the preceding claims, **characterized by** an element (10) which is introduced into the potential cell (2) or into each of the potential and reference cells (2, 3), extends along a longitudinal axis of the measuring chambers (2-1, 3-1) of the measuring cells (2, 3), and is arranged centrally with respect to a cross section of the measuring chambers (2-1, 3-1) of the measuring cells (2, 3) oriented perpendicularly to the longitudinal axis.

7. The tensiometer (1) according to any one of the preceding claims, **characterized in that** the reference system (40) is integrated in the potential cell (2) over its length.

8. The tensiometer (1) according to claim 7, **characterized in that** the reference system (40) forms the element (10) according to claim 6 at least in part.

9. The tensiometer (1) according to any one of the preceding claims, **characterized by** capillary tubes (17) or braids, which are provided with permeable cavities and/or pores (18) and into which the membranes (12) of the measuring cells (2, 3) are each fitted to be flush or are integrated in the cavities at least in part.

10. The tensiometer (1) according to any one of the preceding claims, **characterized by** at least one temperature sensor (8) in or on the potential cell (2) and/or in or on the reference cell (3).

11. The tensiometer (1) according to any one of claims 6 to 10, **characterized in that** the element (10) forms at least a part of the temperature sensor (8) and/or of an electrical contact with the osmotic solution in the potential cell (2), which part is connected to the voltage-measuring apparatus (9).

12. The tensiometer (1) according to any one of claims 9 to 11, **characterized in that** at least the capillary tube (17) or the braid, in which the potential cell (2) is arranged, is electrically conductive at least in part and forms an electrical contact with the (moist) outer membrane surface, to which the voltage-measuring apparatus (9) is connected.

13. The tensiometer (1) according to any one of the preceding claims, **characterized in that** the measuring cells (2, 3) each have an inlet/outlet valve (16) at both ends (14, 15).

14. The tensiometer (1) according to any one of the preceding claims, **characterized by** a concentration-measuring apparatus (13), which is configured to measure at least one concentration difference ($\Delta c$) of a hygroscopic substance within the osmotic solutions (6) in the measuring cells (2, 3).

15. A method for determining a spatially averaged water potential ($\overline{\psi}_w$) in a test piece (20), wherein at least the pressure difference ($\Delta p$) between the measuring chambers (2-1, 3-1) of the potential cell (2) and the reference cell (3), which, due to its arrangement inside the reference chamber (4) of the reference system (40), permits the reference to a selectable reference potential, is measured by means of the tensiometer (1) according to any one of claims 1 to 15, wherein the water and/or matrix potential ($\overline{\psi}_{w/m}$) in the test piece (20), averaged over the length of the potential cell (2), is determined on the basis of the measured pressure difference ($\Delta p$) depending on the semi-permeable membrane used and on the reference potential, and is provided as a measured value.

**Revendications**

1. Tensiomètre (1), comprenant :

   - une cellule de mesure d'une longueur sélectionnable, appelée cellule de potentiel (2), qui comprend une chambre de mesure refermable (2-1) gainée dans une membrane semiperméable tubulaire (12) et qui peut être installée dans une pièce d'essai (20) ;
   - un système de référence (40) comprenant une chambre de référence (4) et une cellule de mesure de longueur sélectionnable, appelée cellule de référence (3), qui comprend une chambre de mesure refermable (3-1) gainée dans une membrane semiperméable tubulaire (12), dans lequel la cellule de référence (3) est intégrée dans la chambre de référence (4) du système de référence (40), et dans lequel la chambre de référence (4) contient une solution de référence osmotique (7) ou peut être remplie d'une telle solution, dans lequel la cellule de potentiel (2) et la cellule de référence (3) sont ou peuvent être remplies d'une solution osmotique (6) ; et
   - un appareil de mesure de pression (5), configuré pour mesurer au moins une différence de pression ($\Delta p$) entre les chambres de mesure (2-1, 3-1) de la cellule de potentiel (2) et la cellule de référence (3).

2. Tensiomètre (1) selon la revendication 1, **caractérisé en ce que** la cellule de référence (3) est structurellement identique à la cellule de potentiel (2).

**3.** Tensiomètre (1) selon la revendication 1, **caractérisé en ce que** le système de référence (40) est plus petit que la cellule de potentiel (2) et est positionné à un endroit approprié dans le tensiomètre (1).

**4.** Tensiomètre (1) selon l'une quelconque des revendications précédentes, **caractérisé en ce que** la chambre de référence (4) comprend un dispositif destiné à définir une pression.

**5.** Tensiomètre (1) selon l'une quelconque des revendications précédentes, **caractérisé par** un appareil de mesure de tension électrique (9) destiné à mesurer une tension électrique (U) entre la face interne et la face externe de la membrane (12) de la cellule de potentiel (2).

**6.** Tensiomètre (1) selon l'une quelconque des revendications précédentes, **caractérisé par** un élément (10) qui est introduit dans la cellule de potentiel (2) ou dans chacune des cellules de potentiel et de référence (2, 3), s'étend le long d'un axe longitudinal des chambres de mesure (2-1, 3-1) des cellules de mesure (2, 3), et est agencé de manière centrale par rapport à une section transversale des chambres de mesure (2-1, 3-1) des cellules de mesure (2, 3) orientée perpendiculairement à l'axe longitudinal.

**7.** Tensiomètre (1) selon l'une quelconque des revendications précédentes, **caractérisé en ce que** le système de référence (40) est intégré dans la cellule de potentiel (2) sur la longueur de la cellule de potentiel.

**8.** Tensiomètre (1) selon la revendication 7, **caractérisé en ce que** le système de référence (40) forme l'élément (10) selon la revendication 6 au moins en partie.

**9.** Tensiomètre (1) selon l'une quelconque des revendications précédentes, **caractérisé par** des tubes capillaires (17) ou des tresses, qui sont pourvus de cavités et/ou de pores perméables (18) et dans lesquels les membranes (12) des cellules de mesure (2, 3) sont ajustées pour affleurer ou intégrées dans les cavités au moins en partie.

**10.** Tensiomètre (1) selon l'une quelconque des revendications précédentes, **caractérisé par** au moins un capteur de température (8) dans ou sur la cellule de potentiel (2) et/ou dans ou sur la cellule de référence (3).

**11.** Tensiomètre (1) selon l'une quelconque des revendications 6 à 10, **caractérisé en ce que** l'élément (10) forme au moins une partie du capteur de température (8) et/ou d'un contact électrique avec la solution osmotique dans la cellule de potentiel (2), connecté à l'appareil de mesure de tension (9).

**12.** Tensiomètre (1) selon l'une quelconque des revendications 9 à 11, **caractérisé en ce qu'**au moins le tube capillaire (17) ou la tresse, dans lequel la cellule de potentiel (2) est agencée, est électriquement conducteur au moins en partie et forme un contact électrique avec la surface de membrane externe (humide), auquel l'appareil de mesure de tension (9) est connecté.

**13.** Tensiomètre (1) selon l'une quelconque des revendications précédentes, **caractérisé en ce que** les cellules de mesure (2, 3) comportent chacune une valve d'entrée/sortie (16) à chacune des deux extrémités (14, 15).

**14.** Tensiomètre (1) selon l'une quelconque des revendications précédentes, **caractérisé par** un appareil de mesure de concentration (13), qui est configuré pour saisir au moins une différence de concentration ($\Delta c$) d'une substance hygroscopique dans les solutions osmotiques (6) dans les cellules de mesure (2, 3).

**15.** Procédé de détermination d'un potentiel d'eau moyenné spatialement ($\overline{\psi_w}$) dans une pièce d'essai (20), dans lequel, au moyen du tensiomètre (1) selon l'une quelconque des revendications 1 à 15, au moins la différence de pression ($\Delta p$) est saisie entre les chambres de mesure (2-1, 3-1) de la cellule de potentiel (2) et la cellule de référence (3) qui, du fait de son agencement à l'intérieur de la chambre de référence (4) du système de référence (40), permet la référence à un potentiel de référence sélectionnable, dans lequel le potentiel de l'eau et/ou de la matrice ($\overline{\psi_{w/m}}$) dans la pièce d'essai (20), moyenné sur la longueur de la cellule de potentiel (2), est déterminé sur la base de la différence de pression ($\Delta p$) saisie et en fonction de la membrane semiperméable utilisée et du potentiel de référence, et est fourni en tant que valeur de mesure.

Fig.1

Fig.2

Fig.3

Fig.4

99

100

100a

101

Fig.5

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- US 5005403 A